# EUROPÄISCHE PATENTSCHRIFT

(11) **EP 4 065 073 B1**
(45) Veröffentlichungstag und Bekanntmachung des Hinweises auf die Patenterteilung: **04.11.2026**
(21) Anmeldenummer: 20781323.9
(22) Anmeldetag: 23.09.2020
(51) Int. Cl.: A61K 8/39, A61K 8/46, A61K 8/86, A61K 8/898, A61Q 5/08, A61Q 5/06

(54) **VERFAHREN ZUR ENTFÄRBUNG VON KERATINISCHEM MATERIAL, DAS DURCH ANWENDUNG EINES PIGMENTS GEFÄRBT WURDE**
METHOD FOR THE DECOLORIZATION OF KERATIN MATERIAL THAT HAS BEEN DYED BY APPLYING A PIGMENT
PROCÉDÉ DE DÉCOLORATION DE MATIÈRE KÉRATINIQUE AYANT ÉTÉ COLORÉE PAR L'APPLICATION D'UN PIGMENT

(30) Priorität: 26.11.2019 DE 102019218227
(43) Veröffentlichungstag der Anmeldung: 05.10.2022
(73) Patentinhaber: Henkel AG & Co. KGaA, 40589 Düsseldorf (DE)
(72) Erfinder: KRUCK, Constanze, 41515 Grevenbroich (DE); HILBIG, Sandra, 44894 Bochum (DE); MOCH, Melanie, 41542 Dormagen (DE); DICKHOF, Susanne, 41748 Viersen (DE); KESSLER-BECKER, Daniela, 51371 Leverkusen (DE)
(86) Internationale Anmeldenummer: PCT/EP2020/076468
(87) Internationale Veröffentlichungsnummer: WO 2021/104709

(56) Entgegenhaltungen:
- WO-A1-2016/091492
- WO-A2-2007/107309
- DE-A1- 102014 218 006
- GB-A- 2 493 207
- GB-A- 2 513 319

## Beschreibung

Die vorliegende Anmeldung liegt auf dem Gebiet der Kosmetik und betrifft ein Verfahren zur Entfärbung von Keratinmaterial, welches durch Anwendung mindestens eines Pigments gefärbt wurde. Das im Rahmen dieses Verfahrens applizierte Entfärbemittel ist gekennzeichnet durch seinen Gehalt an mindestens einem anionischen Tensids und einen pH-Wert, der im Bereich von 1,0 bis 3,5 liegt. Das Entfärbemittel wird auf das gefärbte Keratinmaterial aufgetragen und nach einer Einwirkzeit wieder abgespült. Die vorliegende Anmeldung betrifft ein Verfahren zur Entfärbung von Keratinmaterial, welches durch Anwendung von mindestens einem aminofunktionalisierten Silikonpolymer und mindestens einem Pigment gefärbt wurde.

Die vorliegende Anmeldung betrifft ebenfalls ein Verfahren zum Färben und späteren Entfärben von Keratinmaterial, bei welchem zunächst ein Färbemittel enthaltend mindestens ein aminofunktionalisiertes Silikonpolymer und ein Pigment auf das Keratin aufgetragen wird, und in den nachfolgenden Schritten die Entfärbung durch Anwendung des zuvor beschriebenen Entfärbemittels vorgenommen wird.

Ein Gegenstand der vorliegenden Anmeldung ist eine Mehrkomponenten-Verpackungseinheit, welche in getrennt konfektionierten Containern das Färbe- und das Entfärbemittel enthält. Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren. Ein mögliches, alternatives Färbesystem, das in letzter Zeit zunehmend in den Fokus rückt, beruht auf dem Einsatz von farbigen Pigmenten.

Die Färbung mit Pigmenten bietet verschiedene wesentliche Vorteile. Da die Pigmente sich lediglich von außen an die Keratinmaterialen, insbesondere an die Haarfasern, anlagern, ist die mit dem Färbeprozess verbundene Schädigung ganz besonders gering.

In aktuellen Arbeiten wurde das Problem der geringen Haltbarkeit dieses Färbesystems adressiert. In diesem Zusammenhang konnte gefunden werden, dass die Waschechtheit der mit Pigmenten erhaltenen Farbresultate durch Kombination der Pigmente mit bestimmten aminofunktionalisierten Silikonpolymeren stark verbessert werden konnte.

Durch Einsatz eines geeigneten Entfärbemittels besteht die Möglichkeit, diese Färbungen ohne Beeinflussung der ursprünglichen Haarfarbe des Anwender wieder zu entfernen. Auf diese Weise bietet sich dem Anwender die Option, unmittelbar und ohne großen Aufwand wieder zu seiner Ursprungshaarfarbe zurückkehren zu können. Insbesondere für die Konsumenten, die ihre Haare nicht regelmäßig nachfärben möchten, ist dieser Färbeprozess daher besonders attraktiv.

Ein gut geeignetes Entfärbemittel sollte in der Lage sein, den auf der Oberfläche des Keratinmaterials durch die Anwendung von Pigmenten bzw. Pigmenten und Aminosilikonen ausgebildeten farbigen Film möglichst rückstandsfrei zu entfernen. Wird dieser gefärbte Film nur teilweise bzw. unvollständig entfernt, sind unerwünschte Farbverschiebungen bzw. ein fleckiges Farbergebnis die Folge, die vom Anwender als höchst unattraktiv empfunden werden.

Zur Erhöhung des Anwenderkomforts sollte ein Entfärbemittel die farbigen Filme innerhalb eines möglichst kurzen Zeitraums entfernen, und das Keratinmaterial bzw. die Haare sollten durch Anwendung des Entfärbemittels nicht geschädigt werden. Die Patentdokumente GB 2513319 und JP 2012-167038 beziehen sich beide auf die Entfernung von Farbstoffen aus Haaren.

Aufgabe der vorliegenden Erfindung war die Bereitstellung eines Entfärbemittels zur Entfärbung von gefärbten keratinischen Fasern, welche zuvor durch Anwendung von mindestens einem Pigment, bzw. von mindestens einem aminofunktionalisierten Silikonpolymer und einem Pigment, gefärbt wurden. Hierbei sollte die Entfärbung möglichst vollständig sein, so dass das Keratinmaterial im Idealfall wieder in seinen farblichen Ausgangszustand zurückversetzt werden kann. Des Weiteren sollte die Entfärbung lang anhaltend und gleichmäßig sein, und die entfärbten Keratinfasern sollten weder Nuancenverschiebungen noch Ungleichmäßigkeiten im Farbergebnis erleiden. Zudem sollte das Keratinmaterial durch das Entfärbemittel möglichst wenig geschädigt werden. Überraschenderweise hat sich nun herausgestellt, dass diese Aufgabe in vollumfänglicher Weise gelöst werden kann, wenn keratinisches Material, das zuvor mit mindestens einem Aminosilikon und mit mindestens einem Pigment gefärbt wurde, mit einem Entfärbemittel behandelt wird, welches mindestens ein anionisches Tensid enthält und einen pH-Wert im Bereich von 1,0 bis 3,5 besitzt.

Ein erster Gegenstand der vorliegenden Erfindung ist ein Verfahren zur Entfärbung von Keratinmaterial, welches durch Anwendung mindestens einem aminofunktionalisierten Silikonpolymer und einem Pigment gefärbt wurde, wobei ein Entfärbemittel, welches
(a) mindestens ein anionisches Tensid enthält, und
(b) einen pH-Wert von 1,0 bis 3,5 besitzt,
auf das gefärbte Keratinmaterial aufgetragen und nach einer Einwirkzeit wieder abgespült wird.

Die zu dieser Erfindung führenden Arbeiten haben gezeigt, dass Keratinfasern, insbesondere Haare, durch Anwendung von Pigmenten intensiv gefärbt werden konnten. Ganz besonders intensive Färbeergebnisse wurden hierbei erhalten, wenn die Färbung mit einer Kombination aus Pigment und Aminosilikon erfolgte. Das Pigment bzw. das Gemisch aus Pigment und Aminosilikon lagerte sich in Form eines farbigen Films auf der Oberfläche der Keratinfasern ab. Weiterhin hat sich gezeigt, dass diese Färbungen durch Anwendung des zuvor beschriebenen Entfärbemittels vollständig innerhalb eines kurzen Anwendungszeitraums wieder entfärbt werden konnten, ohne die Haare zu schädigen.

### Entfärbung von keratinischem Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Unter dem Begriff "Mittel zur Entfärbung" wird im Rahmen dieser Erfindung verstanden, dass auf dem Keratinmaterial eine Färbung, die durch Anwendung von mindestens einem Pigment, bzw. mindestens einem aminofunktionalisierten Silikonpolymer und ienem Pigment erzeugt wurde, wieder entfernt werden kann. Bei dieser Färbung wird das Keratinmaterial bzw. die Keratinfaser von einem gefärbten Film umhüllt, der aus den Pigment bzw. dem Pigment und Aminosilikon ausgebildet wird. Die Anwendung des Entfärbemittels erfolgt erfindungsgemäß nach der Anwendung des Färbemittels und ist in der Lage, diesen gefärbten Film wieder von dem Keratinmaterial zu entfernen. Kennzeichnend für das erfindungsgemäße Verfahren ist die Anwendung des Entfärbemittels auf Keratinmaterial, welches zuvor durch Anwendung von mindestens einem Pigment, bzw. mindestens einem Pigment und Aminosilikon, gefärbt wurde.

### aminofunktionalisiertes Silikonpolymer im Färbemittel

Im Rahmen der Erfindung ist ein Verfahren daher dadurch gekennzeichnet, dass das Entfärbemittel auf Keratinmaterial aufgetragen wird, welches durch Anwendung von mindestens einem aminofunktionalisierten Silikonpolymer und mindestens einem Pigment gefärbt wurde.

Das aminofunktionalisiertes Silikonpolymer kann alternativ auch als Aminosilikon oder Amodimethicone bezeichnet werden.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird auch durch die Polymerisationsmethode mitbestimmt. Im Sinne der vorliegenden Erfindung ist es bevorzugt, wenn das maximale Molekulargewicht des Silikonpolymers nicht mehr als 10⁷ g/mol, bevorzugt nicht mehr als 10⁶ g/mol und besonders bevorzugt nicht mehr als 10⁵ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Silikon verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Prinzipiell konnte eine gute Färbeleistung mit aminofunktionalisierten Silikonpolymeren erzielt werden, wenn diese mindestens eine primäre, mindestens eine sekundäre und/oder mindestens eine tertiäre Aminogruppe tragen. Intensive Färbungen mit der besten Waschechtheit wurden jedoch erhalten, wenn ein aminofunktionalisiertes Silikonpolymer im Mittel eingesetzt wurde, welches mindestens eine sekundäre Aminogruppe enthält.

In einer Ausführungsform ist ein erfindungsgemäßes Verfahren dadurch gekennzeichnet, dass das Entfärbemittel auf Keratinmaterial aufgetragen wird, welches durch Anwendung von mindestens einem aminofunktionalisierten Silikonpolymer mit mindestens einer sekundären Aminogruppe gefärbt wurde.

Die sekundäre(n) Aminogruppe(n) kann bzw. können sich an verschiedenen Positionen des aminofunktionalisierten Silikonpolymers befinden. Ganz besonders gute Farbresultate wurden erhalten, wenn ein aminofunktionalisiertes Silikonpolymer eingesetzt wurde, dass mindestens eine, bevorzugt mehrere Struktureinheiten der Formel (Si-Amino) besitzt.

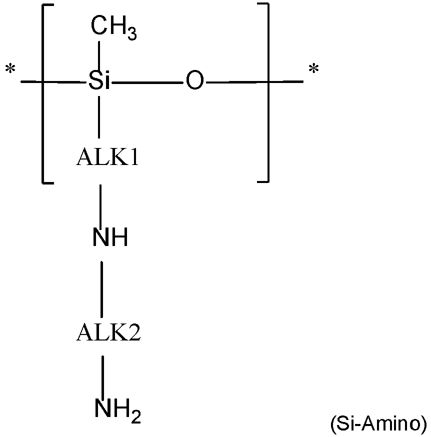

In den Struktureinheiten der Formel (Si-Amino) stehen die Kürzel ALK1 und ALK2 unabhängig voneinander für eine lineare oder verzweigte, zweiwertige C₁-C₂₀-Alkylengruppe.

In einer weiteren ganz besonders bevorzugten Ausführungsform ist ein erfindungsgemäßes Verfahren dadurch gekennzeichnet, dass das Entfärbemittel auf Keratinmaterial aufgetragen wird, welches durch Anwendung von mindestens einem aminofunktionalisierten Silikonpolymer gefärbt wurde, welches mindestens eine Struktureinheit der Formel (Si-Amino) umfasst, 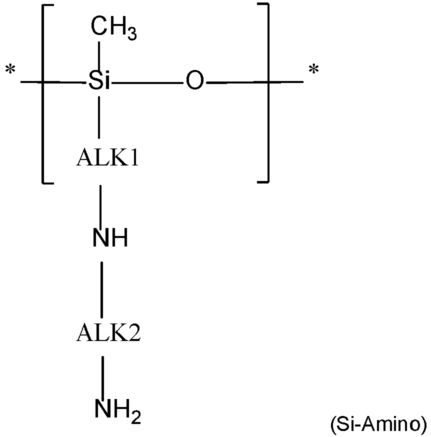 wobei
- ALK1 und ALK2: unabhängig voneinander für eine lineare oder verzweigte, zweiwertige C₁-C₂₀-Alkylengruppe stehen.

Die mit einem Stern (*) gekennzeichneten Positionen geben hierbei jeweils die Bindung zu weiteren Struktureinheiten des Silikonpolymers an. Beispielsweise kann das dem Stern benachbarte SiliciumAtom an ein weiteres Sauerstoffatom gebunden sein, und das dem Stern benachbarte Sauerstoffatom kann an ein weiteres Siliciumatom oder auch an eine C₁-C₆-Alkylgruppe gebunden sein.

Eine zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite Bindung besteht zwischen ALK2 und der primären Aminogruppe.

Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In einer weiteren besonders bevorzugten Ausführungsform stellen die Struktureinheiten der Formel (Si-Amino) Wiederholungseinheiten im aminofunktionalisierten Silikonpolymer dar, so dass das Silikonpolymer mehrere Struktureinheiten der Formel (Si-Amino) umfasst.

Im Folgenden werden besonders gut geeignete aminofunktionalisierte Silikonpolymere mit mindestens einer sekundären Aminogruppe aufgelistet.

Färbungen mit den allerbesten Waschechtheiten konnten erhalten werden, wenn bei der vorangehenden Färbung mindestens ein Mittel auf dem keratinischen Material appliziert wurde, das mindestens ein aminofunktionalisiertes Silikonpolymer enthält, das Struktureinheiten der Formel (Si-I) und der Formel (Si-II) umfasst

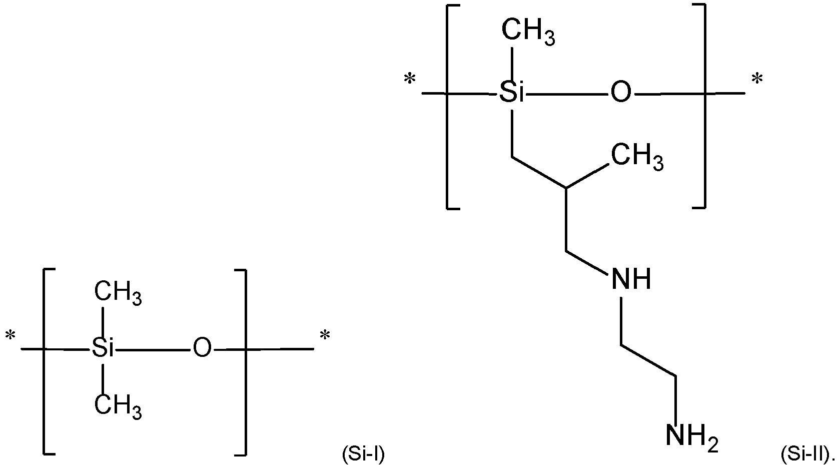

In einer weiteren explizit ganz besonders bevorzugten Ausführungsform ist ein erfindungsgemäßes Verfahren dadurch gekennzeichnet, dass das Entfärbemittel auf Keratinmaterial aufgetragen wird, welches durch Anwendung von mindestens einem aminofunktionalisierten Silikonpolymer gefärbt wurde,welches Struktureinheiten der Formel (Si-I) und der Formel (Si-II) umfasst

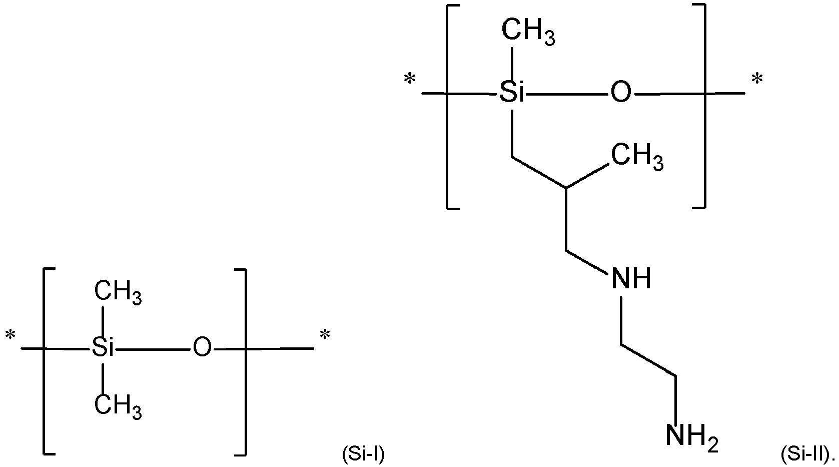

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt. Ein weiteres besonders bevorzugtes Handelsprodukt ist Dowsil AP-8658 Amino Fluid, das ebenfalls von der Firma Dow Chemical Company kommerziell vertrieben wird.

Im Rahmen einer weiteren bevorzugten Ausführungsform kann die Anwendung des Entfärbemittel auch auf Keratinmaterial erfolgen, welches zuvor durch die Anwendung eines Färbemittels gefärbt wurde, das mindestens ein aminofunktionelles Silikonpolymer der Formel der Formel (Si-III) enthält, 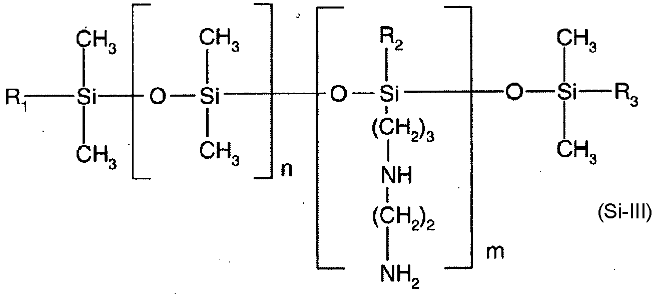 wobei
- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Verfahren sind gekennzeichnet durch die vorangehende Anwendung eines Färbemittels auf dem keratinischem Material, wobei das Färbemittel mindestens aminofunktionelles Silikonpolymer der Formel der Formel (Si-IV) enthält, 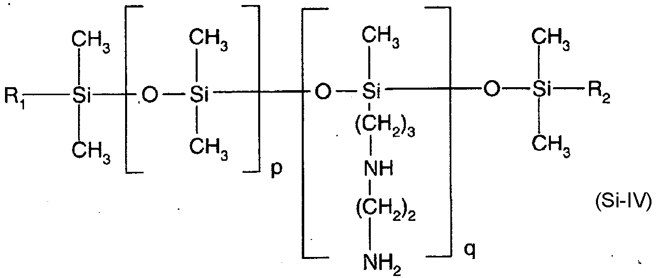 in der
- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-Si(CH₃)₂-Gruppe an eine -[O-Si(CH₃)₂]-Gruppierung gebunden.

Als besonders wirkungsvoll im Hinblick auf die Erzeugung von intensiven Farbresultaten haben sich auch erfindungsgemäßen Verfahren erwiesen, in welchen ein Färbemittel auf den Keratinfasern appliziert wird, welches mindestens ein aminofunktionelles Silikonpolymer der Formel der Formel (Si-V) enthält 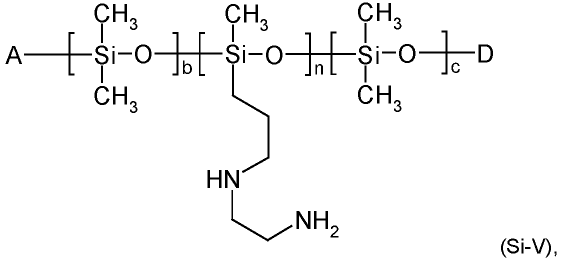 in der
- A: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- b, n und c: für ganze Zahlen zwischen 0 und 1000 stehen, mit den Maßgaben

- n > 0 und b + c > 0
- mindestens eine der Bedingungen A = -OH bzw. D = -H ist erfüllt.

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das zuvor applzierte Färbemittel kann weiterhin auch ein oder mehrere verschiedene aminofunktionalisierte Silikonpolymere enthalten, die durch die Formel (Si-VI)

M(RₐQ_{b}SiO_{(4-a-b)/2)x}(R_{c}SiO_{(4-c)/2)y}M (Si-VI)

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist -N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer besonders bevorzugten Ausführungsform ist ein erfindungsgemäßes Verfahren gekennzeichnet durch die vorangehende Anwendung eines Färbemittels auf dem keratinischem Material, wobei das Färbemittel ein aminofunktionelles Silikonpolymer der Formel (Si-VII)

R'ₐG₃₋ₐ-Si(OSiG ₂)ₙ-(OSiG _{b}R'_{2- b})ₘ-O-SiG₃₋ₐ-R'ₐ (Si-VII),

enthält, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, - CH₂CH₂CH₂CH₃, -O-CH₂CH(CH₃)₂, -CH₂CH(CH₃)₂, -O-CH(CH₃)CH₂CH₃, - CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N⁺(R")₃A⁻
   ∘ -Q-N⁺H(R")₂A⁻
   ∘ -Q-N⁺H₂(R")A⁻
   ∘ -Q-N(R")-CH₂-CH₂-N⁺R"H₂A⁻,
   wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
   R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein erfindungsgemäßes Verfahren gekennzeichnet durch die vorangehende Anwendung eines Färbemittels auf dem keratinischem Material, wobei das Färbemittel mindestens ein aminofunktionelles Silikonpolymer der Formel (Si-Vlla) enthält, 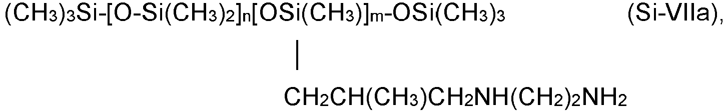 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Verfahren gekennzeichnet durch die vorangehende Anwendung eines Färbemittels auf dem keratinischem Material, wobei das Färbemittel mindestens ein aminofunktionelles Silikonpolymer der Formel (Si-Vllb) enthält 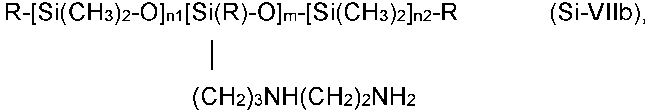 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Färbemittel bevorzugt, die ein aminofunktionelles Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Weiterhin sind auch Färbemittel zum Einsatz im erfindungsgemäßen Verfahren geeignet, welche ein spezielles 4-Morpholinomethyl-substituiertes Silikonpolymer enthielten. Dieses aminofunktionalisierte Silikonpolymer umfasst Struktureinheiten der Formeln (SI-VIII) und der Formel (Si-IX)

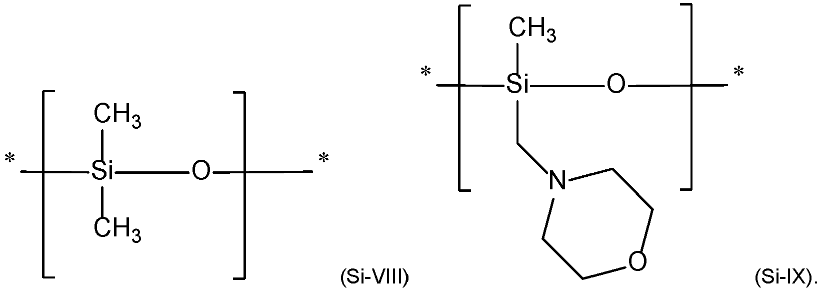

Entsprechende 4-Morpholinomethyl-substituiertes Silikonpolymere werden im folgenden beschrieben.

Ein entsprechendes aminofunktionalisiertes Silikonpolymer ist unter dem Namen Amodimethicone/Morpholinomethyl Silsesquioxane Copolymer bekannt und in Form des Rohstoffes Belsil ADM 8301 E von Wacker kommerziell erhältlich.

Als 4-morpholinomethyl-substituiertes Silikon kann beispielsweise ein Silikon eingesetzt werden, welches Struktureinheiten der Formeln (Si-VIII), (Si-IX) und (Si-X) aufweist 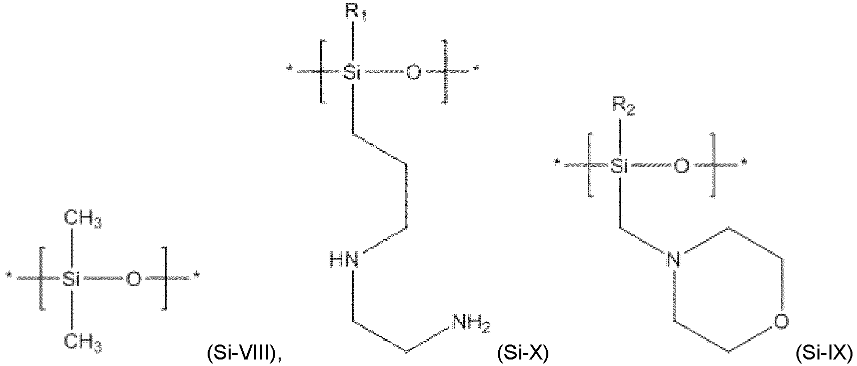 in denen
- R1: für -CH₃, -OH, -OCH₃, -O-CH₂CH₃, -O-CH₂CH₂CH₃, oder -O-CH(CH₃)₂ steht;
- R2: für -CH₃, -OH, oder -OCH₃ steht.

Besonders bevorzugte Färbemittel enthalten mindestens ein 4-morpholinomethyl-substituierten Silikons der Formel (Si-XI) 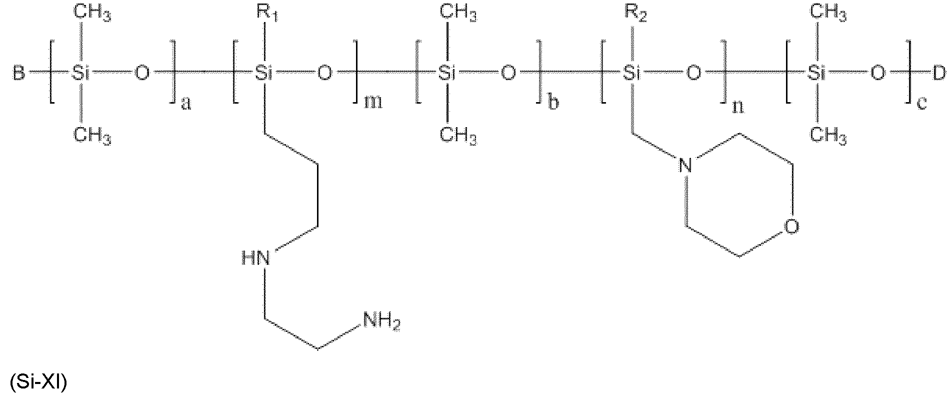 in der
R1 für -CH₃, -OH, -OCH₃, -O-CH₂CH₃, -O-CH₂CH₂CH₃, oder -O-CH(CH₃)₂ steht;
R2 für -CH₃, -OH, oder -OCH₃ steht.
B für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht,
a, b und c unabhängig voneinander für ganze Zahlen zwischen 0 und 1000 stehen, mit der Maßgabe a + b + c > 0
m und n unabhängig voneinander für ganze, Zahlen zwischen 1 und 1000 stehen mit den Maßgabe, daß
   - mindestens eine der Bedingungen B = -OH bzw. D = -H erfüllt ist,
   - die Einheiten a, b, c, m und n statistisch oder blockweise im Molekül verteilt vorliegen.

Strukturformel (Si-XI) soll verdeutlichen, daß die Siloxangruppen n und m nicht zwingend direkt an eine Endgruppierung B bzw. D gebunden sein müssen. Vielmehr gilt in bevorzugten Formeln (Si-VI) a > 0 oder b > 0 und in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.

Die durch Formel (Si-VI) dargestellten erfindungsgemäß eingesetzten Silikone können trimethylsilylterminiert sein (D oder B = -Si(CH₃)₃), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | | |
|---|---|---|
| B = -O-Si(CH₃)₂OH und | D = -Si(CH₃)₃ | |
| B = -O-Si(CH₃)₂OH und | D = -Si(CH₃)₂OH | |
| B = -O-Si(CH₃)₂OH und | D = -Si(CH₃)₂OCH₃ | |
| B = -O-Si(CH₃)₃ | und | D = -Si(CH₃)₂OH |
| B = -O-Si(CH₃)₂OCH₃ | und | D = -Si(CH₃)₂OH |

bedeutet. Diese Silikone führen zu exorbitanten Verbesserungen der Haareigenschaften der mit den erfindungsgemäßen Mitteln behandelten Haare, und zu einem gravierend verbesserten Schutz bei oxidativer Behandlung.

Bei dem im erfindungsgemäßen Verfahren zur vorangehenden Färbung eingesetzten Mittel können ein oder mehrere aminofunktionalisierte Silikonpolymeren beispeilsweise in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthalten. Hierbei sind die Mengenangaben auf die Gesamtmenge aller eingesetzten Aminosilikone bezogen, die zum Gesamtgewicht des Färbemittels in Relation gesetzt wird.

In diesem Zusammenhang wurde beobachtet, dass der Eindruck von fettigen bzw. öligen Haaren auch von der Einsatzmenge an Aminosilikonen abhängt. Je höher der Gehalt an Aminosilikon im Färbemittel war, desto negativer war auch der haptische Eindruck. Der Einsatz des erfindungsgemäßen Entfärbemittels zeigte eine besonders starke Verbesserung des Haargefühls, wenn bei der vorangehenden Anwendung des Färbmittels ein sehr hoher Gewichtsanteil an Aminosilikonen eingesetzt wurde.

Im Rahmen einer weiteren besonders bevorzugten Ausführungsform ist ein erfindungsgemäßes Verfahren dadurch gekennzeichnet, dass das Färbemittel - bezogen auf das Gesamtgewicht des Mittels - ein oder mehrere aminofunktionalisierte Silikonpolymere in einer Gesamtmenge von 0,1 bis 8,0 Gew.-%, bevorzugt 0,4 bis 5,0 Gew.-%, weiter bevorzugt von 0,8 bis 3,0 Gew.-% und ganz besonders bevorzugt von 1,0 bis 3,5 Gew.-% enthält.

### Pigmente im Färbemittel

Im erfindungsgemäßen Verfahren wird ein Entfärbemittel auf Keratinmaterial appliziert, das zuvor durch Anwendung mindestens eines Pigments gefärbt wurde.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein. In einer bevorzugten Ausführungsform ist ein erfindungsgemäßes Verfahren dadurch gekennzeichnet, dass dass das Entfärbemittel auf Keratinmaterial aufgetragen wird, welches durch Anwendung von mindestens einem anorganischen und/oder organischen Pigment gefärbt wurde.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer bevorzugten Ausführungsform ist ein erfindungsgemäßes Verfahren dadurch gekennzeichnet, dass das Entfärbemittel auf Keratinmaterial aufgetragen wird, welches durch Anwendung von mindestens einem anorganischen Pigment gefärbt wurde, wobei das anorganische Pigment bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer bevorzugten Ausführungsform ist ein erfindungsgemäßes Verfahren dadurch gekennzeichnet, dass das Entfärbemittel auf Keratinmaterial aufgetragen wird, welches durch Anwendung von mindestens einem Pigment gefärbt wurde, das ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona^{®} sind beispielsweise:
Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das zuvor applizierte Färbemittel auch ein oder mehrere organischen Pigmente enthalten.

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren, dadurch gekennzeichnet, dass das Entfärbemittel auf Keratinmaterial aufgetragen wird, welches durch Anwendung von mindestens einem organischen Pigment gefärbt wurde, wobei das organische Pigment bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Mittel ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Bei dem im erfindungsgemäßen Verfahren zur vorangehenden Färbung eingesetzten Mittel können ein oder mehrere Pigmente beispeilsweise in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthalten. Hierbei sind die Mengenangaben auf die Gesamtmenge aller eingesetzten Pigmente bezogen, die zum Gesamtgewicht des Färbemittels in Relation gesetzt wird.

In einer weiteren ganz besonders bevorzugten Ausführungsform ist ein erfindungsgemäßes Färbemittel dadurch gekennzeichnet, dass das Färbemittel - bezogen auf das Gesamtgewicht des Färbemittels - ein oder mehrere Pigmente in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

Als weiteren optionalen Bestanteil könnten die Färbemittel zusätzlich auch noch auch einen oder mehrere direktziehende Farbstoffe enthalten. Bei direktziehenden Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L.

Direktziehende Farbstoffe können in anionische, kationische und nichtionische direktziehende Farbstoffe unterteilt werden.

In einer weiteren Ausführungsform kann ein erfindungsgemäßes Mittel dadurch gekennzeichnet sein, dass es zusätzlich mindestens eine farbgebende Verbindung aus der Gruppe der anionischen, nichtionischen und kationischen direktziehenden Farbstoffe enthält.

Geeignete kationische direktziehende Farbstoffe sind beispielsweise Basic Blue 7, Basic Blue 26, HC Blue 16, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76.

Als nichtionische direktziehende Farbstoffe können beispielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO⁻, -SO₃⁻ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Im Rahmen einer weiteren Ausführungsform kann ein Mittel zum Färben von keratinischem Material dadurch gekennzeichnet sein, dass es mindestens einen anionischen direktziehenden Farbstoff enthält, der ausgewählt ist aus der Gruppe der Nitrophenylendiamine, der Nitroaminophenole, der Azofarbstoffe, der Anthrachinonfarbstoffe, der Triarylmethanfarbstoffe, der Xanthen-Farbstoffe, der Rhodamin-Farbstoffe, der Oxazinfarbstoffen und/oder der Indophenolfarbstoffe, wobei die Farbstoffe aus der vorgenannten Gruppe jeweils mindestens eine Carbonsäuregruppe (-COOH), eine Natriumcarboxylatgruppe (-COONa), eine Kaliumcarboxylatgruppe (-COOK), eine Sulfonsäuregruppe (-SO₃H) eine Natriumsulfonatgruppe (-SO₃Na) und/oder eine Kaliumsulfonatgruppe (-SO₃K) besitzen.

Als geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1),

Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%.

Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

In einer weiteren Ausführungsform ist ein erfindungsgemäßes Färbemittel daher dadurch gekennzeichnet, dass es mindestens einen direktziehenden Farbstoff enthält, der ausgewählt ist aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Der oder die direktziehenden Farbstoffe können je nach erwünschter Farbintensität in verschiedenen Mengen im Färbemittel eingesetzt werden. Gute Ergebnisse konnten erhalten werden, wenn das Färbemittel - bezogen auf das Gesamtgewicht des Färbemittel - ein oder mehrere direktziehende Farbstoffe in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthält.

Weiterhin kann das Mittel als zusätzlichen optionalen Bestandteil auch noch eine farbgebende Verbindung aus der Gruppe der photochromen oder thermochromen Farbstoff enthalten.

Photochrome Farbstoffe sind Farbstoffe, die auf Bestrahlung mit UV-Licht (Sonnenlicht oder Schwarzlicht) mit einer reversiblen Farbtonänderung reagieren. Dabei verändert das UV-Licht die chemische Struktur der Farbstoffe und damit ihr Absorptionsverhalten (Photochromie).

Thermochrome Farbstoffe sind Farbstoffe, die auf Temperaturänderungen mit einer reversiblen Farbtonänderung reagieren. Dabei verändert die Temperaturänderung die chemische Struktur der Farbstoffe und damit ihr Absorptionsverhalten (Thermochromie).

Das Färbemittel kann - bezogen auf das Gesamtgewicht des Färbemittels - ein oder mehrere photochrome und/oder thermochrome Farbstoffe in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthalten.

### Entfärbemittel

Im Rahmen des erfindungsgemäßen Verfahrens wird auf das Keratinmaterial, insbesondere auf menschliche Haare, das bzw. die wie zuvor beschrieben gefärbt wurden, ein Entfärbemittel appliziert. Das Entfärbemittel wird auf das gefärbte Keratinmaterial aufgetragen und nach einer Einwirkzeit wieder abgespült.

Der Zeitpunkt, zu dem das Entfärbemittel angewendet wird, hängt von den Bedürfnissen das Anwenders ab und kann an dessen Gewohnheiten angepasst werden.

Beispielsweise ist es möglich, dass Entfärbemittel auf das frisch gefärbte, noch nasse oder bevorzugt getrocknete Keratinmaterial aufzutragen, so dass zwischen dem Ausspülen des Färbemittels und der Anwendung des Entfärbemittels ein Zeitraum von einigen Stunden liegt. Diese Anwendung des Entfärbemittels kurz nach der Färbung kann insbesondere dann vorgenommen werden, wenn das Ergebnis der Färbung nicht mit den Erwartungen des Anwenders übereinstimmt. Auch ist es beispielsweise möglich, eine sehr intensive oder auffällige Färbung zu einem bestimmten Anlass vorzunehmen und die Färbung nach diesem Anlass wieder zu entfernen.

Ebenso ist es möglich, dass zwischen der vorangehenden Anwendung des Färbemittels und der Anwendung des Entfärbemittels ein längerer Zeitraum liegt, der bei einigen bis mehreren Tagen oder sogar Wochen liegen kann. In diesem Zusammenhang gilt die Voraussetzung, dass das Entfärbemittel auf gefärbtes Keratinmateral aufgetragen wird, was bedeutet, dass das Keratinmaterial immer noch durch die Anwendung der Pigmente gefärbt sein muss.

Das Entfärbemittel ist dadurch gekennzeichnet, dass es
(a) mindestens ein anionisches Tensid enthält, und
(b) einen pH-Wert von 1,0 bis 3,5 besitzt.

### Anionische Tenside (a) im Entfärbemittel

Als wesentlichen Inhaltssstoff enthält das erfindungsgemäße Entfärbemittel mindestens ein anionisches Tensid (a).

Unter dem Begriff Tenside (T) werden grenzflächenaktive Substanzen, die an Ober- und Grenzflächen Adsorptionsschichten bilden oder in Volumenphasen zu Mizell-Kolloiden oder lyotropen Mesophasen aggregieren können, verstanden. Man unterscheidet anionische Tenside bestehend aus einem hydrophoben Rest und einer negativ geladenen hydrophilen Kopfgruppe, amphotere bzw. zwitterionische Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Zu den geeigneten anionischen Tensiden (a), die im erfindungsgemäßen Entfärbemittel eingesetzt werden können, zählen:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel R-(OCH₂-CH₂)ₓ-OSO₃⁻ X⁺, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, 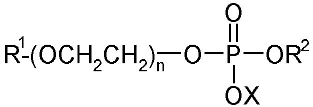 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder NR³R⁴R⁵R⁶, mit R³ bis R⁶ unabhängig voneinander stehend für einen C₁ bis C₄ -Kohlenwasserstoffrest, steht.

Ganz besonders gute Ergebnisse wurden erhalten, wenn im Entfärbemittel mindestens ein anionischesTensid (a) der Formel (T-1) eingesetzt wurde, 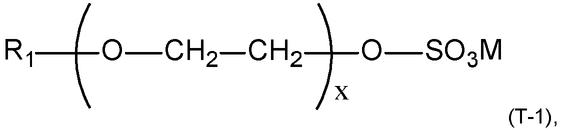 wobei
- R¹: für eine lineare oder verzweigte, gesättigte oder ungesättigte C₈-C₃₀-Alkylgruppe steht,
- x: für eine ganze Zahl von 0 bis 50 steht, und
- M: für ein Wasserstoffatom, für Ammonium (NH₄)₊ oder für ein Äquivalent eines ein oder mehrwertigen Kations steht.

Im Rahmen einer ganz besonders bevorzugten Ausführungsform ist ein erfindungsgemäßes Verfahren dadurch gekennzeichnet, dass das Entfärbemittel mindestens ein anionischesTensid (a) der Formel (T-1) enthält, 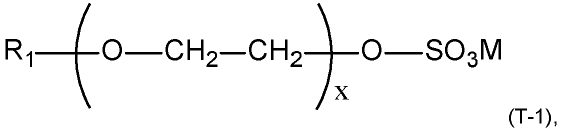 wobei
- R₁: für eine lineare oder verzweigte, gesättigte oder ungesättigte C₈-C₃₀-Alkylgruppe steht,
- x: für eine ganze Zahl von 0 bis 50 steht, und
- M: für ein Wasserstoffatom, für Ammonium (NH₄)₊ oder für ein Äquivalent eines ein oder mehrwertigen Kations steht.

Der Rest R1 stellt den hydrophoben Teil des anionischen Tensids dar und steht für eine lineare oder verzweigte, gesättigte oder ungesättigte C₈-C₃₀-Alkylgruppe. Wenn der Rest für eine ungesättigte C₈-C₃₀-Alkylgruppe steht, dann kann die Alkylgruppe einfach oder mehrfach ungesättigt sein.

Bevorzugt steht der Rest R1 für eine lineare, gesättigte oder ungesättigte C₈-C₃₀-Alkylgruppe- Ganz besonders bevorzugt steht der Rest R1 für eine lineare, gesättigte oder ungesättigte C₁₂-C₂₂-Alkylgruppe. Explizit ganz besonders bevorzugt steht der Rest R1 für eine R für eine lineare, gesättigte oder ungesättigte C₁₂-C₁₈-Alkylgruppe.

Beispiele für gesättigte, lineare C₈-C₃₀-Alkylgruppen sind die Laurylgruppe, die Myristylgruppe, die Cetylgruppe, die Stearylgruppe und die Behenylgruppe.

Die Indexzahl x gibt die Anzahl der im anionischen Tensid enthatlenen Ethylenoxid-Gruppen an. Steht x für die Zahl 0, dann besitzt das anionische Tensid der Formel (T-1) keine EthylenoxidEinheiten, in diesem Fall liegt ein Alkyl-Sulfat bzw. das Salz eines Alkyl-Sulfats vor.

Beispiele für die Salzs von Alkylsulfaten sind Natriumlaurylsulfat und Natriummyristylsulfat.

Bevorzugt steht x für eine ganze Zahl von 0 bis 5, besonders bevorzugt steht x für eine ganze Zahl von 1 bis 5.

Der Rest M steht für ein Wasserstoffatom, für Ammonium (NH₄)₊ oder für ein Äquivalent eines ein oder mehrwertigen Kations.

Steht M für ein Wasserstoffatom, dann liegt das anionische Tensid in Form des protonierten (und gegebenenfalls ethoxylierten) Schwefelsäureesters vor. In wässriger Lösung steht die protonierte Form mit der deprotonierten Form im Gleichgewicht, wobei die deprotonierte Form eine anionische Ladung trägt. Aus diesem Grund fallen auch die protonierten Verbindungen der Formel (I) in die Gruppe der anionischen Tenside.

Steht M für Ammonium (NH₄)₊ oder für ein Äquivalent eines ein oder mehrwertigen Kations, dann liegt das anionsiche Tensid der Formel (I) in Form seines Salzes vor. Das Vorhandensein der entsprechenden Äquivalent eines ein- oder mehrwertigen Kations gewährleistet hierbei die Elektroneutralität des Anion-Tensid. Bevorzugt steht M für ein einwertiges Kation, insbesondere für ein Natrium- oder Kalium-Kation.

Im Rahmen einer ganz besonders bevorzugten Ausführungsform ist ein erfindungsgemäßes Verfahren dadurch gekennzeichnet, dass das Entfärbemittel mindestens ein anionisches Tensid (a) der Formel (T-1) enthält, wobei
- R1: für eine lineare, gesättigte oder ungesättigte C₁₂-C₁₈-Alkylgruppe steht,
- x: für eine ganze Zahl von 0 bis 5, bevorzugt für eine ganze Zahl von 1 bis 5, steht,
- M: für ein Wasserstoffatom oder für ein Alkalimetallkation, bevorzugt für ein Natriumkation oder für ein Kaliumkation, steht.

Ein ganz besonders bevorzugtes anionisches Tensid der Formel (I) kann beispielsweise unter dem Handelsnamen Texapon NSO BZ (BZ = preserved with benzoic acid) und der INCI Bezeichnung Sodium Laureth Sulfate von der Firma BASF kommerziell bezogen werden. Dieses Sodium Laureth Sulfate besitzt die CAS-Nummer 68891-38-3.

Weiterhin konnte ein Entfärbemittel mit guter Entfärbeleistung erhalten werden, wenn das erfindungsgemäße Entfärbmittel mindestens ein anionisches Tensid (a) der Formel (T-2) enthielt, 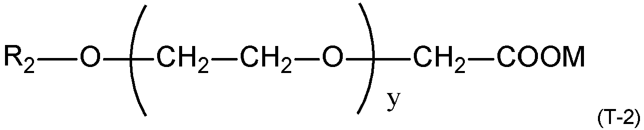 wobei
- R2: für eine lineare oder verzweigte, gesättigte oder ungesättigte C₈-C₃₀-Alkylgruppe steht,
- y: für eine ganze Zahl von 0 bis 16 steht,
- M: für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

Im Rahmen einer weiteren bevorzugten Ausführungsform ist ein erfindungsgemäßes Verfahren dadurch gekennzeichnet, dass das Entfärbemittel mindestens ein anionisches Tensid (a) der Formel (T-2) enthält, 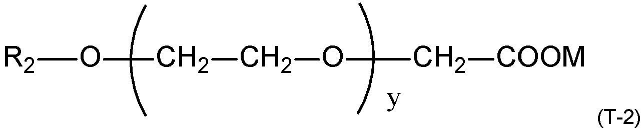 wobei
- R2: für eine lineare oder verzweigte, gesättigte oder ungesättigte C₈-C₃₀-Alkylgruppe steht,
- y: für eine ganze Zahl von 0 bis 16 steht,
- M: für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

Der Rest R2 stellt den hydrophoben Teil des anionischen Tensids der Formtel (T-2) dar und steht für eine lineare oder verzweigte, gesättigte oder ungesättigte C₈-C₃₀-Alkylgruppe. Wenn der Rest für eine ungesättigte C₈-C₃₀-Alkylgruppe steht, dann kann die Alkylgruppe einfach oder mehrfach ungesättigt sein.

Bevorzugt steht der Rest R2 für eine lineare, gesättigte oder ungesättigte C₈-C₃₀-Alkylgruppe- Ganz besonders bevorzugt steht der Rest R2 für eine lineare, gesättigte oder ungesättigte C₁₂-C₂₂-Alkylgruppe. Explizit ganz besonders bevorzugt steht der Rest R2 für eine R für eine lineare, gesättigte oder ungesättigte C₁₂-C₁₈-Alkylgruppe.

Beispiele für gesättigte, lineare C₈-C₃₀-Alkylgruppen sind die Laurylgruppe, die Myristylgruppe, die Cetylgruppe, die Stearylgruppe und die Behenylgruppe.

Die Indeyzahl y gibt die Anzahl der im anionischen Tensid enthaltenen Ethylenoyid-Gruppen an. Steht y für die Zahl 0, dann besitzt das anionische Tensid der Formel (T-2) keine EthylenoxidEinheiten, in diesem Fall liegt ein Alkyl-Sulfat bzw. das Salz eines Alkyl-Sulfats vor.

Bevorzugt steht y für eine ganze Zahl von 0 bis 6, besonders bevorzugt steht y für eine ganze Zahl von 1 bis 6.

Der Rest M steht für ein Wasserstoffatom, für Ammonium (NH₄)₊ oder für ein Äquivalent eines ein oder mehrwertigen Kations.

Steht M für ein Wasserstoffatom, dann liegt das anionische Tensid in Form des protonierten (und gegebenenfalls ethoxylierten) Schwefelsäureesters vor. In wässriger Lösung steht die protonierte Form mit der deprotonierten Form im Gleichgewicht, wobei die deprotonierte Form eine anionische Ladung trägt. Aus diesem Grund fallen auch die protonierten Verbindungen der Formel (I) in die Gruppe der anionischen Tenside.

Steht M für Ammonium (NH₄)₊ oder für ein Äquivalent eines ein oder mehrwertigen Kations, dann liegt das anionsiche Tensid der Formel (I) in Form seines Salzes vor. Das Vorhandensein der entsprechenden Äquivalent eines ein- oder mehrwertigen Kations gewährleistet hierbei die Elektroneutralität des Anion-Tensid. Bevorzugt steht M für ein einwertiges Kation, insbesondere für ein Natrium- oder Kalium-Kation.

Ein Beispiel für ein besonders gut geeignets anionisches Tensid der Formel (T-2) ist Sodium Laureth-6 Carboxylate, das unter dem Handelsnamen Akypo Soft 45 HP von der Firma Kao kommerziell bezogen werdenkann. Das Tensid wird auch als Alkyl ether carboxylic acid sodium salt bezeichnet und trägt die CAS-Nummer 33939-64-9.

Zur Gewährleistung besonders guter Farbresultate im erfindungsgemäßen Verfahren enthält das Entfärbemittel das bzw. die anionischen Tenside (a) bevorzugt in bestimmten Mengenbereichen. Besonders intensive und gleichmäßige Färbungen konnten erhalten werden, wenn das Entfärbemittel- bezogen auf das Gesamtgewicht des Entfärbemittels - ein oder mehrere anionischeTenside (a) in einer Gesamtmenge von 1,0 bis 15,0 Gew.-%, bevorzugt 1,5 bis 13,0 Gew.-%, weiter bevorzugt 3,0 bis 12,0 Gew.-% und ganz besonders bevorzugt 6,0 bis 11,0 Gew.-% enthält.

Im Rahmen einer ganz besonders bevorzugten Ausführungsform ist ein erfindungsgemäßes Verfahren dadurch gekennzeichnet, dass das Entfärbemittel - bezogen auf das Gesamtgewicht des Entfärbemittels - ein oder mehrere anionischeTenside (a) in einer Gesamtmenge von 1,0 bis 15,0 Gew.-%, bevorzugt 1,5 bis 13,0 Gew.-%, weiter bevorzugt 3,0 bis 12,0 Gew.-% und ganz besonders bevorzugt 6,0 bis 11,0 Gew.-% enthält.

### Wassergehalt im Entfärbemittel

Da das Entfärbemittel erfindungsgemäß auf einen sauren pH-Wert im Bereich von 1,0 bis 3,5 eingestellt wird, enthält es Wasser bzw. umfasst einen wasserhaltigen Träger.

Demzufolge ist ein erster Gegenstand der vorliegenden Erfindung mit anderen Worten ein Verfahren zur Entfärbung von Keratinmaterial, welches durch Anwendung mindestens eines Pigments gefärbt wurde, wobei ein Entfärbemittel, welches
(a) mindestens ein anionisches Tensid enthält, und
(b) Wasser enthält und einen pH-Wert von 1,0 bis 3,5 besitzt,
auf das gefärbte Keratinmaterial aufgetragen und nach einer Einwirkzeit wieder abgespült wird.

Eine besonders gute Entfernung der überschüssigen Pigmente bzw. Aminosilikone war möglich, wenn das Entfärbemittel - bezogen auf das Gesamtgewicht des Entfärbemittels -einen Wassergehalt von 50 bis 99 Gew.-%, bevorzgut von 55 bis 98 Gew.-%, weiter bevorzugt von 60 bis 97 Gew.-%, und besonders bevorzugt von 70 bis 96 Gew.-% besaß.

In einer weiteren besonders bevorzugten Ausführungsform ist ein erfindungsgemäßes Verfahren daher dadurch gekennzeichnet, dass das Entfärbemittel - bezogen auf das Gesamtgewicht des Entfärbemittels - einen Wassergehalt von 50 bis 99 Gew.-%, bevorzgut von 55 bis 98 Gew.-%, weiter bevorzugt von 60 bis 97 Gew.-%, und besonders bevorzugt von 70 bis 96 Gew.-% besitzt.

### pH-wert des Entfärbemittel

Kennzeichnend für das Entfärbemittel ist ein saurer pH-Wert im Bereich von 1,0 bis 3,5.

In der zu dieser Erfindung führenden Reihenversuchen hat sich gezeigt, dass durch die Wahl des optimalen pH-Wertes die Entfärbeleistung gesteuter werden konnte. Eine gute Entfärbewirkung konnte bereits ab einem pH-Wert von 3,5 beobachtet werden. Durch ein weiteres Absenken des pH-Wertes konnte diese Entfärbeleistung jedoch noch weiter verbessert werden.

In diesem Zusammenhang hat es sich als ganz besonders bevorzugt herausgestellt, wenn das Entfärbemittel einen pH-Wert (b) von 1,5 bis 3,3, bevorzugt von 1,8 bis 3,0 und besonders bevorzugt von 2,0 bis 3,0 besitzt.

Im Rahmen einer weiteren ganz besonders bevorzugten Ausführungsform ist ein erfindungsgemäßes Verfahren dadurch gekennzeichnet, dass das Entfärbemittel einen pH-Wert (b) von 1,5 bis 3,3, bevorzugt von 1,8 bis 3,0 und besonders bevorzugt von 2,0 bis 3,0 besitzt.

### Auftrag des Entfärbemittels

Innerhalb des erfindungsgemäßen Verfahrens wird das Entfärbemittel auf das gefärbte Keratinmaterial aufgetragen und nach einer Einwirkzeit wieder abgespült.

Da das Entfärbemittel auf das gefärbte Haar aufgetragen wird, muss das Entfärbemittel nach der Anwendung des zuvor beschriebenen Färbemittels auf das Keratinmaterial appliziert werden.

Anders ausgedrückt wird das Entfärbemittel auf dem Keratinmaterial angwendet, nachdem das Färbemittel ausgespült und das Keratinmaterial bevorzugt zur genauen Bestimmung des Farbergebnisses getrocknet wurde.

Der genaue Zeitpunkt der Anwendung des Entfärbemittels bestimmt sich hierbei nach dem Wunsch des Anwenders, die unerwünschte oder nicht mehr benötigte Färbung wieder zu entfernen. So kann beispielsweise das Entfärbemittel 12 bis 24 Stunden nach Anwendung des Färbemittels auf das gefärbte Keratinmaterial appliziert werden. Im Rahmen einer weiteren Ausführungsform kann der Anwender die gefärbten Keratinmatieralien, insbesondere die Haare, aber auch über einen Zeitraum von mehreren Tagen bis Wochen tragen, bis er beschließt, die Färbung wieder zu verändern bzw. seine Ursprungshaarfarbe wieder zurück haben möchte.

### weitere Tenside im Entfärbemittel

Zusätzlich zu den zuvor beschriebenen zwitterionischen bzw. amphoteren Tensiden (a) kann das erfindungsgemäße Entfärbemittel optional zusätzlich auch noch weitere Tenside enthalten.

In einer weiteren ganz besonders bevorzugten Ausführungsform ist ein erfindungsgemäßes Verfahren dadurch gekennzeichnet, dass das Entfärbemittel mindestens ein kationisches, nichtionisches und/oder zwitterionisches bzw. amphoteres Tensid enthält.

Unter dem Begriff Tenside (T) werden grenzflächenaktive Substanzen, die an Ober- und Grenzflächen Adsorptionsschichten bilden oder in Volumenphasen zu Mizell-Kolloiden oder lyotropen Mesophasen aggregieren können, verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind
- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Nichtionische Tenside enthalten beipsielsweise als hydrophile Gruppe eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®} - Typen (Cognis),
- alkoxilierte Triglyceride,
- alkoxilierte Fettsäurealkylester der Formel (Tnio-1)

   R¹CO-(OCH₂CHR²)_{w}OR³ (Tnio-1)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (Tnio-2)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (Tnio-2) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

- Zuckertenside vom Typ der Fettsäure-N-alkylpolyhydroxyalkylamide, ein nichtionisches Tensid der Formel (Tnio-3),

   R⁵CO-NR⁶-[Z] (Tnio-3)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (Tnio-4) wiedergegeben werden:

   R⁷CO-(NR⁸)-CH₂-[CH(OH)]₄-CH₂OH (Tnio-4)

   Vorzugsweise werden als Fettsäure-N-alkylpolyhydroxyalkylamide Glucamide der Formel (Tnio-4) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (Tnio-4), die durch reduktive Aminierung von Glucose mit Methylamin und anschließende Acylierung mit Laurinsäure oder C12/14-Kokosfettsäure beziehungsweise einem entsprechenden Derivat erhalten werden. Weiterhin können sich die Polyhydroxyalkylamide auch von Maltose und Palatinose ableiten.

Weitere typische Beispiele für nichtionische Tenside sind Fettsäureamidpolyglycolether, Fettaminpolyglycolether, Mischether bzw. Mischformale, Proteinhydrolysate (insbesondere pflanzliche Produkte auf Weizenbasis) und Polysorbate.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure sowie die Zuckertenside erwiesen. Zubereitungen mit hervorragenden Eigenschaften werden ebenfalls erhalten, wenn sie als nichtionische Tenside Fettsäureester von ethoxyliertem Glycerin enthalten.

Bevorzugte zwitterionische Tenside sind beispielsweise Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

### weitere optionale Inhaltsstoffe im Entfärbemittel

Zusätzlich zu den bereits beschriebenen erfindungswesentlichen Bestandteilen kann das Entfärbemittel auch noch weitere optionale Inhaltsstoffe enthalten, wie beispielsweise Lösungsmittel, anionische, nichtionische, zwitterionische und/oder kationische Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP-und Styrol/Acrylamid-Copolymere; Perlglanzmittel wie Ethylenglykolmono- und -distearat sowie PEG-3-distearat; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Anwendung des Entfärbemittels

Im Rahmen des erfindungsgemäßen Verfahrens wird das zuvor beschriebene Entfärbemittel auf das gefärbte Keratinmaterial aufgetragen und nach einer Einwirkzeit wieder abgespült.

Die Anwendung kann beispielsweise mit der (behandschuten) Hand oder aber mit Hilfe eines Applikators, wie beispeilsweise eines Pinsels oder einer Aplicette, oder aber auch einer Bürste, oder eines Kamms erfolgen.

Abhängig davon, ob der Anwender eine vollständige Entfärbung wünscht, oder ob nur bestimmte Partien bwz. Strähnen entfärbt werden sollen, kann das Entfärbemittel entweder auf dem gesamten keratinischen Material (wie beispielsweise dem gesamten Kopfhaar) oder aber auf bestimmte Teile oder entsprechende Strähnen des Keratinmaterials oder der Keratinfasern aufgetragen werden.

Nach dem Applizieren bzw. Auftragen wird das Entfärbemittel für einen bestimmten Zeitraum auf das Keratinmaterial einwirken gelassen. Beispielsweise kann eine Einwirkzeit von 5 bis 60 Minuten, bevorzugt von 5 bis 30 Minuten, weiter bevorzugt von 5 bis 15 Minuten und ganz besonders bevorzugt von 5 bis 10 Minuten gewählt werden. Nach dieser Einwirkzeit wird das Entfärbemittel wider mit Wasser ausgespült

Im Rahmen einer weiteren bevorzugten Ausführungsform ist ein erfindungsgemäßes Verfahren dadurch gekennzeichnet, dass das Entfärbemittel auf das gefärbte Keratinmaterial aufgetragen und nach einer Einwirkzeit von 5 bis 60 Minuten, bevorzugt von 5 bis 30 Minuten, weiter bevorzugt von 5 bis 15 Minuten und ganz besonders bevorzugt von 5 bis 10 Minuten wieder abgespült wird.

Das Einwirken des Entfärbemittels auf das Keratinmaterial kann bei Raumtemperatur bzw. bei der Körpertemperatur vorgenommen werden. Zur Unterstützung oder Beschleungung des Farbabzug kann das mit dem Entfärbemittel beaufschlagte Keratinmaterial jedoch auch erhöhten Temperaturen ausgesetzt werden. Es ist erfindungsgemäß, wenn das Entfärbemittel auf das gefärbte Keratinmaterial aufgetragen wir das Keratinmaterial während des Einwirkens des Entfärbemittels auf eine Temperatur von 25 bis 70 °, bevorzugt von 25 bis 60 °C, weiter bevorzugt von 30 bis 55 °C und ganz besonders bevorzugt von 40 bis 55 °C erhitzt wird.

Im Rahmen einer weiteren b Ausführungsform ist ein erfindungsgemäßes Verfahren dadurch gekennzeichnet, dass
- das Entfärbemittel auf das gefärbte Keratinmaterial aufgetragen wird
- das Keratinmaterial während des Einwirkens des Entfärbemittels auf eine Temperatur von 25 bis 70 °, bevorzugt von 25 bis 60 °C, weiter bevorzugt von 30 bis 55 °C und ganz besonders bevorzugt von 40 bis 55 °C erhitzt wird, und dann
- das Entfärbemittel wieder abgespült wird.

Neben der thermischen Unterstützung des Entfärbeprozesses ist es darüberhinaus auch möglich, das mit dem Entfärbemittel beaufschlagte Keratinmaterial einer mechanischen Beanspruchung auszusetzen, um die Ablösung des bei der Färbung auf dem Keratinmaterial ausgebildeten Films zu verbessern. So kann das Keratinmaterial während des Entfärbevorgangs beispielsweise mit den Händen massiert oder mit einem Kamm oder einer Bürste gekämmte werden. Auch jede andere machanische Beanspruchung, die geeignet ist, unter Einwirkung des Entfärbemittels die Ablösung des gefärbten Films vom Keratinmaterial zu verbessern, ist denkbar und vom erfindungsgemäßen Verfahren umasst.

Im Rahmen einer weiteren bevorzugten Ausführungsform ist ein erfindungsgemäßes Verfahren dadurch gekennzeichnet, dass
- das Entfärbemittel auf das gefärbte Keratinmaterial aufgetragen wird,
- das Keratinmaterial während des Einwirkens des Entfärbemittels gekämmt, massiert, gebürstet oder auf eine andere Weise einer mechanischen Krafteinwirkung ausgesetzt wird, und dann
- das Entfärbemittel wieder abgespült wird.

Wie bereits zuvor beschrieben, kann das erfindungsgemäße Entfarbemittel angewendet werden, um Keratinmaterial, welches durch Anwendung eines Pigments, bzw. mindestens eines Pigments und mindestens eines Aminosilikons, gefärbt wurde, zu entfärben. Stellt der Anwender zum Beispiel nach der Färbung fest, dass das Farbergebnis nicht seinen Wünschen entspricht, so kann er dies zum Anlass nehmen, die Färbung durch Anwendung des Entfärbemittels wider zu entfernen.

Weiterhin kann der Anwender eine Färbung und die spätere Entfärbung auch von vorneherein planen, beispielsweise, wenn er seine Haare zu einem bestimmten Anlass färben und danach wieder entfärben möchte. Zu diesem Zweck können dem Anwender auch alle Mittel bzw. Formuleirungen, die sowohl für die Färbung als auch für die Entfärbung notwendig sind, zur Verfügung gestellt werden.

Ein zweiter Gegenstand der vorliegenden Erfindung ist daher ein Verfahren zum Färben und späteren Entfärben von menschlichen Haaren, umfassend die folgenden Schritte:
(1) Auftragen eines Färbemittels auf die Haare, wobei das Färbemittel mindestens ein aminofunktionalisiertes Silikonpolymer und mindestens ein Pigment enthält, wie sie bei der Beschreibung des ersten Erfindungsgegenstands bereits im Detail offenbart wurden,
(2) Einwirkenlassen des Färbemittels auf die Haare,
(3) Ausspülen des Färbemittels aus den Haaren,
(4) Auftragen eines Entfärbemittels auf die Haare, wobei das Entfärbemittel bei der Beschreibung des ersten Erfindungsgegenstand im Detail offenbart wurde,
(5) Einwirkenlassen des Entfärbemittels auf die Haare,
(6) Ausspülen des Entfärbemittels aus den Haaren.

Ein weiterer Gegenstand der vorliegenden Erfindung ist daher ein Verfahren zum Färben und späteren Entfärben von menschlichen Haaren, umfassend die folgenden Schritte:
(1) Auftragen eines Färbemittels auf die Haare, wobei das Färbemittel mindestens ein aminofunktionalisiertes Silikonpolymer und mindestens ein Pigment enthält, wie sie bei der Beschreibung des ersten Erfindungsgegenstands bereits im Detail offenbart wurden,
(2) Einwirkenlassen des Färbemittels auf die Haare,
(3) Ausspülen des Färbemittels aus den Haaren,
(4) Auftragen eines Entfärbemittels auf die Haare, wobei das Entfärbemittel bei der Beschreibung des ersten Erfindungsgegenstand im Detail offenbart wurde,
(5) Einwirkenlassen des Entfärbemittels auf die Haare,
(6) Ausspülen des Entfärbemittels aus den Haaren.

Die Pigmente und die aminofunktionalisierten Silikonpolymere wurden bereits bei der Beschreibung des ersten Erfindungsgegenstands im Detail offenbart. Auch das Entfärbemittel wurde bereits bei der Beschreibung des ersten Erfindungsgegenstands im Detail offenbart.

### Mehrkomponenten-Verpackungseinheit

Für den Anwender ist es besonders komfortabel, wenn ihm die entsprechenden Färbe- und Entfärbemittel in Form einer Mehrkomponenten-Verpackungseinheit zur Verfügung gestellt werden.

Ein weiterer Gegentstand der vorliegenden Erfindung ist daher eine Mehrkomponenten-Verpackungseinheite (Kit-of-parts) zur Färbung und Entfärbung von Keratinmaterial, umfassend getrennt voneinander konfektioniert:
- einen ersten Container mit einem Färbemittel, wobei das Färbemittel mindestens ein aminofunktionalisiertes Silikonpolymer und mindestens ein Pigment enthält, wie sie bei der Beschreibung des ersten Erfindungsgegenstands bereits im Detail offenbart wurden, und
- einen zweiten Container mit einem Entfärbemittel, wobei das Entfärbemittel bei der Beschreibung des ersten Erfindungsgegenstand im Detail offenbart wurde.

Betreffend die weiteren bevorzugten Ausführungsformen der erfindungsgemäßen Mehrkomponenten-Verpackungseinheit und der Verwendung gilt *mutatis mutantis* das zum erfindungsgemäßen Verfahren gesagte.

### Beispiele

### 1. Formulierungen

Es wurden die folgenden Formulierungen hergestellt (alle Angaben, sofern nichts anderes angegeben ist, in Gew.-%)

| Färbemittel (FM) | FM (Gew.-%) |
|---|---|
| Cetylalkohol | 3,0 |
| Stearylalkohol | 3,0 |
| Ceteareth-30, (Cetearylalkohol, ethoxylated 30 EO) | 1,5 |
| Kaliumdihydrogenphosphat | 0,35 |
| Dinatriumhydrogenphosphat | 0,72 |
| Unipure Red LC3071, organisches Pigment CI 15850 | 1,0 |
| 1,2-Propandiol | 10,0 |
| Phenoxyethanol | 0,8 |
| Dow Corning 2-8566 (Siloxanes and Silicones, 3-[(2-Aminoethyl)amino]-2-methylpropyl Me, Di-Me-Siloxane | 1,0 |
| Natriumsalicylat | 0,4 |
| Wasser | ad 100 |

| Entfärbemittel (EM) (Gew.-%) | EM-1 Vergleich | EM-2 Vergleich | EM-3 Erfindung |
|---|---|---|---|
| Natriumlaurethsulfat (C12-C14, ethoxyliert mit 2 EO) | 8,7 | 7,0 | 14,0 |
| Dinatriumcocoamphodiacetat | 2,0 | --- | --- |
| Laureth-6-carboxylat, Natriumsalz | -- | --- | --- |
| Natronlauge/Zitronensäure | ad pH 4,6 | ad pH 4,7 | ad pH 2,9 |
| Wasser | ad 100 | ad 100 | ad 100 |

| Entfärbemittel (EM) (Gew.-%) | EM-4 Erfindung | EM-5 Erfindung | EM-6 Erfindung |
|---|---|---|---|
| Natriumlaurethsulfat (C12-C14, ethoxyliert mit 2 EO) | --- | --- | --- |
| Dinatriumcocoamphodiacetat | --- | --- | --- |
| Laureth-6-carboxylat, Natriumsalz | 10,0 | 10,0 | 10,0 |
| Cocoamidopropylbetain | --- | --- | --- |
| Dimethylcocoylbetain | --- | --- | --- |
| Natronlauge/Zitronensäure | ad pH 2,9 | ad pH 2,4 | ad pH 2,1 |
| Wasser | ad 100 | ad 100 | ad 100 |

### 2. Anwendung

Nach der Herstellung wurde das Färbemittel (FM) auf Haarsträhnen (Kerling, Euronaturhaar weiß) appliziert. Das Färbemittel wurde für drei Minuten einwirken gelassen. Im Anschluss daran wurden die Haarsträhne gründlich (1 Minute) mit Wasser ausgewaschen, getrocknet und für 24 Stunden ruhen glassen.

Eine der gefärbten Strähnen wurde mit einem Farbmessgerät der Firma Datacolor, Typ Spectraflash 450 vermessen.

Das Entfärbemittel wurde jeweils auf eine gefärbte Haarsträhne aufgetragen, für 5 Minuten einmassiert und dann mit Wasser ausgespült. Die auf diese Weise entfärbten Haare wurden trocknen gelassen und dann farbmetrisch vermessen.

Der für die Beurteilung des Farberhalts herangezogene dE-Wert ergibt sich aus den am jeweiligen Strähnenteil gemessenen L*a*b*-Farbmesswerten wie folgt: $dE = {\left[{\left({L}_{i}-{L}_{0}\right)}^{2}+{\left({a}_{i}-{a}_{0}\right)}^{2}+\left({b}_{i}-{b}_{0}\right)\right]}^{1 / 2}$
L₀, a₀ und b₀ = Messwerte der ungefärbten Strähne
Lᵢ, aᵢ und bᵢ = Messwerte der gefärbten/entfärbten Strähne

Je kleiner der dE-Wert ist, desto geringer ist der Farbabstand im Vergleich zur ungefärbten Strähne und desto besser ist die Entfärbewirkung.

| | L | a | b | dE | Entfärbeleistung |
|---|---|---|---|---|---|
| Haarsträhne Euronaturhaar weiß, uncoloriert | 73,18 | 2,35 | 22,09 | --- | --- |
| Färbung mit (FM) | 41,43 | 47,38 | 4,98 | --- | --- |
| Färbung mit (FM) und Entfärbung mit (EM-1) | 40,61 | 46,83 | 12,14 | 56,0 | schlecht |
| Färbung mit (FM) und Entfärbung mit (EM-2) | 39,0 | 48,95 | 3,55 | 60,7 | schlecht |
| Färbung mit (FM) und Entfärbung mit (EM-3) | 49,33 | 32,85 | 6,36 | 41,8 | mittel |
| Färbung mit (FM) und Entfärbung mit (EM-4) | 62,84 | 27,05 | 8,30 | 30,1 | gut |
| Färbung mit (FM) und Entfärbung mit (EM-5) | 57,29 | 27,45 | 6,84 | 33,4 | gut |
| Färbung mit (FM) und Entfärbung mit (EM-6) | 59,65 | 26,72 | 9,48 | 30,6 | gut |

| | | | | | |
|---|---|---|---|---|---|
| dE = Farbabstand zum unbehandelten Haar | | | | | |

## Patentansprüche

1. Verfahren zur Entfärbung von Keratinmaterial, welches durch Anwendung von mindestens einem aminofunktionalisierten Silikonpolymer und mindestens einem Pigment gefärbt wurde, wobei ein Entfärbemittel, welches
(a) mindestens ein anionisches Tensid enthält, und
(b) einen pH-Wert von 1,0 bis 3,5 besitzt,
auf das gefärbte Keratinmaterial aufgetragen und nach einer Einwirkzeit wieder abgespült wird.

2. Verfahren nach Anspruch 1, **dadurch gekennzeichnet, dass** das Entfärbemittel auf Keratinmaterial aufgetragen wird, welches durch Anwendung von mindestens einem aminofunktionalisierten Silikonpolymer mit mindestens einer sekundären Aminogruppe gefärbt wurde.

3. Verfahren nach einem der Ansprüche 1 bis 2, **dadurch gekennzeichnet, dass** das Entfärbemittel auf Keratinmaterial aufgetragen wird, welches durch Anwendung von mindestens einem aminofunktionalisierten Silikonpolymer gefärbt wurde, welches mindestens eine Struktureinheit der Formel (Si-Amino) umfasst, 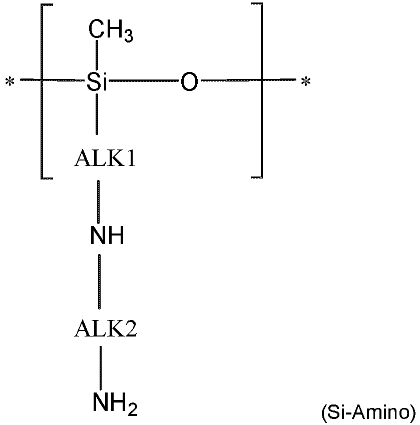 wobei
ALK1 und ALK2 unabhängig voneinander für eine lineare oder verzweigte, zweiwertige C₁-C₂₀-Alkylengruppe stehen.

4. Verfahren nach einem der Ansprüche 1 bis 3, **dadurch gekennzeichnet, dass** das Entfärbemittel auf Keratinmaterial aufgetragen wird, welches durch Anwendung von mindestens einem aminofunktionalisierten Silikonpolymer gefärbt wurde,welches Struktureinheiten der Formel (Si-I) und der Formel (Si-II) umfasst

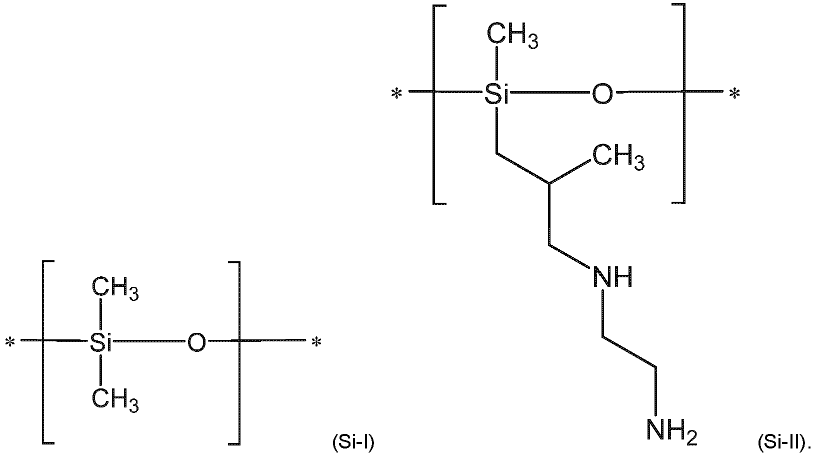

5. Verfahren nach einem der Ansprüche 1 bis 4, **dadurch gekennzeichnet, dass** das Entfärbemittel auf Keratinmaterial aufgetragen wird, welches durch Anwendung von mindestens einem anorganischen Pigment gefärbt wurde, wobei das anorganische Pigment bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

6. Verfahren nach einem der Ansprüche 1 bis 5, **dadurch gekennzeichnet, dass** das Entfärbemittel auf Keratinmaterial aufgetragen wird, welches durch Anwendung von mindestens einem organischen Pigment gefärbt wurde, wobei das organische Pigment bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

7. Verfahren nach einem der Ansprüche 1 bis 6, **dadurch gekennzeichnet, dass** das Entfärbemittel mindestens ein anionischesTensid (a) der Formel (T-1) enthält, 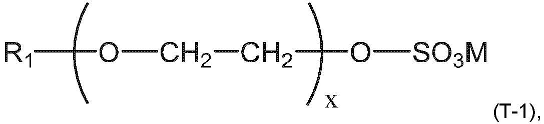 wobei
R₁ für eine lineare oder verzweigte, gesättigte oder ungesättigte C₈-C₃₀-Alkylgruppe steht,
x für eine ganze Zahl von 0 bis 50 steht, und
M für ein Wasserstoffatom, für Ammonium (NH₄)₊ oder für ein Äquivalent eines ein oder mehrwertigen Kations steht.

8. Verfahren nach einem der Ansprüche 1 bis 7, **dadurch gekennzeichnet, dass** das Entfärbemittel mindestens ein anionisches Tensid (a) der Formel (T-2) enthält, 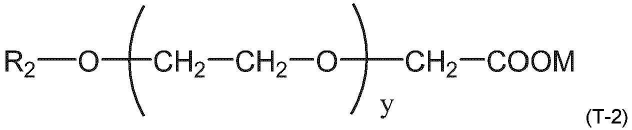 wobei
R2 für eine lineare oder verzweigte, gesättigte oder ungesättigte C₈-C₃₀-Alkylgruppe steht,
y für eine ganze Zahl von 0 bis 16 steht,
M für Wasserstoff oder ein Metall wie ein Alkalimetall, insbesondere Natrium, Kalium, Lithium, ein Erdalkalimetall, insbesondere ½ Magnesium, ½ Calcium, ½ Zink, oder ein Ammoniumion (NH4⁺) steht.

9. Verfahren nach einem der Ansprüche 1 bis 8, **dadurch gekennzeichnet, dass** das Entfärbemittel - bezogen auf das Gesamtgewicht des Entfärbemittels - ein oder mehrere anionischeTenside (a) in einer Gesamtmenge von 1,0 bis 15,0 Gew.-%, bevorzugt 1,5 bis 13,0 Gew.-%, weiter bevorzugt 3,0 bis 12,0 Gew.-% und ganz besonders bevorzugt 6,0 bis 11,0 Gew.-% enthält.

10. Verfahren nach einem der Ansprüche 1 bis 9, **dadurch gekennzeichnet, dass** das Entfärbemittel einen pH-Wert (b) von 1,5 bis 3,3, bevorzugt von 1,8 bis 3,0 und besonders bevorzugt von 2,0 bis 3,0 besitzt.

11. Verfahren nach einem der Ansprüche 1 bis 10, **dadurch gekennzeichnet, dass** das Entfärbemittel auf das gefärbte Keratinmaterial aufgetragen und nach einer Einwirkzeit von 5 bis 60 Minuten, bevorzugt von 5 bis 30 Minuten, weiter bevorzugt von 5 bis 15 Minuten und ganz besonders bevorzugt von 5 bis 10 Minuten wieder abgespült wird.

12. Verfahren zum Färben und späteren Entfärben von menschlichen Haaren, umfassend die folgenden Schritte:
(1) Auftragen eines Färbemittels auf die Haare, wobei das Färbemittel mindestens ein aminofunktionalisiertes Silikonpolymer und mindestens ein Pigment enthält, wie sie in den Ansprüchen 1 bis 6 definiert wurden,
(2) Einwirkenlassen des Färbemittels auf die Haare,
(3) Ausspülen des Färbemittels aus den Haaren,
(4) Auftragen eines Entfärbemittels auf die Haare, wobei das Entfärbemittel in den Ansprüchen 1 bis 10 definiert wurde,
(5) Einwirkenlassen des Nachbehandlungsmittels auf die Haare,
(6) Ausspülen des Nachbehandlungsmittels aus den Haaren.

13. Mehrkomponenten-Verpackungseinheit (Kit-of-parts) zur Färbung und Entfärbung von Keratinmaterial, umfassend getrennt voneinander konfektioniert:
- einen ersten Container mit einem Färbemittel, wobei das Färbemittel mindestens ein aminofunktionalisiertes Silikonpolymer und mindestens ein Pigment enthält, wie sie in den Ansprüchen 1 bis 6 definiert wurden, und
- einen zweiten Container mit einem Entfärbemittel, wobei das Entfärbemittel in den Ansprüchen 1 bis 10 definiert wurde.

## Claims

1. Method for decolorizing keratin material that has been colored by applying at least one amino-functionalized silicone polymer and at least one pigment, wherein a decolorizing agent that
(a) contains at least one anionic surfactant, and
(b) has a pH of 1.0 to 3.5,
is applied to the colored keratin material and rinsed off after a processing time.

2. Method according to claim 1, **characterized in that** the decoloring agent is applied to keratin material that has been colored by applying at least one amino-functionalized silicone polymer having at least one secondary amino group.

3. Method according to any one of claims 1 to 2, **characterized in that** the decolorizing agent is applied to keratin material that has been colored by the use of at least one amino-functionalized silicone polymer comprising at least one structural unit of the formula (Si-Amino), 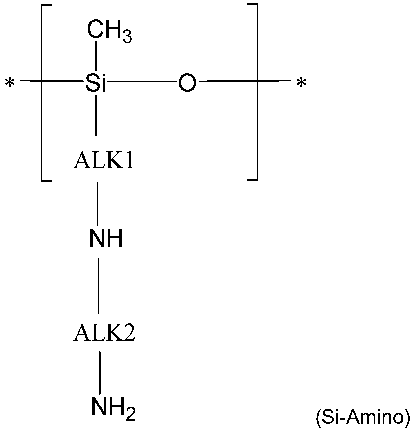 wherein
ALK1 and ALK2 independently represent a linear or branched, divalent C₁-C₂₀ alkylene group.

4. Method according to any one of claims 1 to 3, **characterized in that** the decolorizing agent is applied to keratin material that has been colored by applying at least one amino-functionalized silicone polymer comprising structural units of the formula (Si-I) and the formula (Si-II)

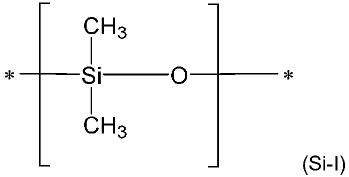

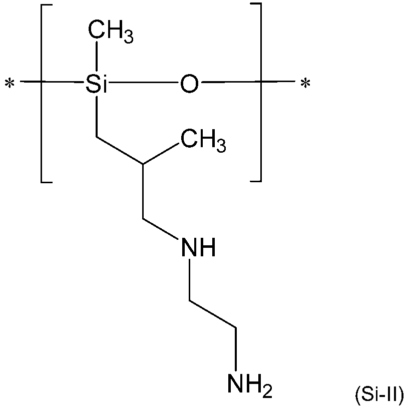

5. Method according to any one of claims 1 to 4, **characterized in that** the decoloring agent is applied to keratin material that has been colored by the use of at least one inorganic pigment, wherein the inorganic pigment is preferably selected from the group consisting of colored metal oxides, metal hydroxides, metal oxide hydrates, silicates, metal sulfides, complex metal cyanides, metal sulfates, bronze pigments, and/or colored pigments based on mica or glimmer that are coated with at least one metal oxide and/or a metal oxychloride.

6. Method according to any one of claims 1 to 5, **characterized in that** the decolorizing agent is applied to keratin material that has been dyed by applying at least one organic pigment, wherein the organic pigment is preferably selected from the group consisting of carmine, quinacridone, phthalocyanine, sorghum, blue pigments with the Color Index numbers CI 42090, CI 69800, CI 69825, CI 73000, CI 74100, CI 74160, yellow pigments with the Color Index numbers CI 11680, CI 11710, CI 15985, CI 19140, CI 20040, CI 21100, CI 21108, CI 47000, CI 47005, green pigments with the Color Index numbers CI 61565, CI 61570, CI 74260; orange pigments with Color Index numbers CI 11725, CI 15510, CI 45370, CI 71105; red pigments with Color Index numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, and/or CI 75470.

7. Method according to any one of claims 1 to 6, **characterized in that** the decolorizing agent contains at least one anionic surfactant (a) of the formula (T-1), 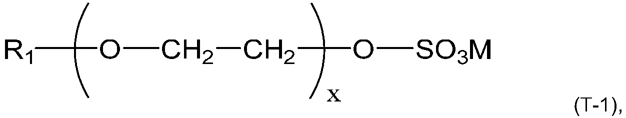 where
R₁ represents a linear or branched, saturated or unsaturated C₈-C₃₀ alkyl group,
x represents an integer from 0 to 50, and
M represents a hydrogen atom, ammonium (NH₄)₊, or an equivalent of a monovalent or polyvalent cation.

8. Method according to any one of claims 1 to 7, **characterized in that** the decolorizing agent comprises at least one anionic surfactant (a) of the formula (T-2), 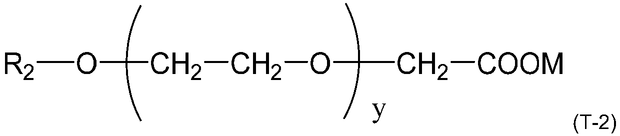 wherein
R2 represents a linear or branched, saturated or unsaturated C₈-C₃₀ alkyl group,
y represents an integer from 0 to 16,
M represents hydrogen or a metal such as an alkali metal, in particular sodium, potassium, lithium, an alkaline earth metal, in particular ½ magnesium, ½ calcium, ½ zinc, or an ammonium ion (NH4⁺).

9. Method according to any one of claims 1 to 8, **characterized in that** the decolorizing agent - based on the total weight of the decolorizing agent - contains one or more anionic surfactants (a) in a total amount of 1.0 to 15.0 wt.%, preferably 1.5 to 13.0 wt.%, more preferably 3.0 to 12.0 wt.%, and most preferably 6.0 to 11.0 wt.%.

10. Method according to any one of claims 1 to 9, **characterized in that** the decolorizing agent has a pH (b) of 1.5 to 3.3, preferably 1.8 to 3.0, and most preferably 2.0 to 3.0.

11. Method according to any one of claims 1 to 10, **characterized in that** the decolorizing agent is applied to the dyed keratin material and, after an exposure time of 5 to 60 minutes, preferably 5 to 30 minutes, more preferably 5 to 15 minutes, and most preferably 5 to 10 minutes, is rinsed off.

12. Method for dyeing and subsequently decolorizing human hair, comprising the following steps:
(1) Applying a dye to the hair, wherein the dye contains at least one amino-functionalized silicone polymer and at least one pigment as defined in claims 1 to 6,
(2) Allowing the dye to act on the hair,
(3) Rinsing the dye out of the hair,
(4) Applying a decolorizing agent to the hair, wherein the decolorizing agent is as defined in claims 1 to 10,
(5) Allowing the after-treatment agent to act on the hair,
(6) Rinsing the after-treatment agent out of the hair.

13. Multi-component packaging unit (kit-of-parts) for coloring and decoloring keratinous material, comprising the following, packaged separately from one another:
- a first container containing a coloring agent, wherein the coloring agent contains at least one amino-functionalized silicone polymer and at least one pigment as defined in claims 1 to 6, and
- a second container containing a decolorizing agent, wherein the decolorizing agent is defined in claims 1 to 10.

## Revendications

1. Procédé de décoloration d'une matière kératinique qui a été colorée par l'application d'au moins un polymère de silicone à fonction amino et d'au moins un pigment, dans lequel un agent de décoloration, qui
(a) contient au moins un tensioactif anionique, et
(b) présente un pH compris entre 1,0 et 3,5,
est appliqué sur la matière kératinique colorée puis rincé après un temps de pose.

2. Procédé selon la revendication 1, **caractérisé en ce que** l'agent de décoloration est appliqué sur une matière kératinique qui a été colorée par l'application d'au moins un polymère de silicone à fonction amino comportant au moins un groupe amino secondaire.

3. Procédé selon l'une des revendications 1 à 2, **caractérisé en ce que** l'agent décolorant est appliqué sur une matière kératinique qui a été colorée par l'utilisation d'au moins un polymère de silicone à fonction amino, comprenant au moins un motif structural de formule (Si-amino), 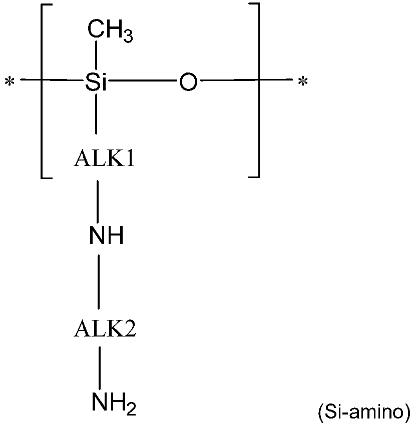 où
ALK1 et ALK2 représentent, indépendamment l'un de l'autre, un groupe alkylène en C₁ à C₂₀ divalent, linéaire ou ramifié.

4. Procédé selon l'une des revendications 1 à 3, **caractérisé en ce que** l'agent décolorant est appliqué sur une matière kératinique qui a été colorée par l'application d'au moins un polymère de silicone à fonction amino, comprenant des motifs structuraux de formule (Si-I) et de formule (Si-II)

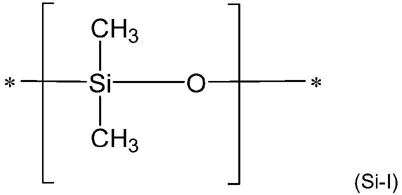

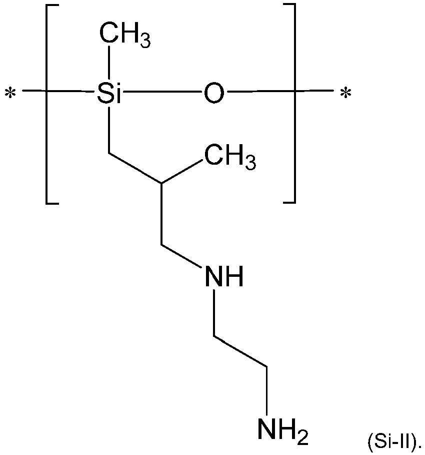

5. Procédé selon l'une des revendications 1 à 4, **caractérisé en ce que** l'agent décolorant est appliqué sur une matière kératinique qui a été colorée par l'application d'au moins un pigment inorganique, le pigment inorganique étant de préférence choisi parmi le groupe des oxydes métalliques colorés, des hydroxydes métalliques, des hydrates d'oxydes métalliques, silicates, des sulfures métalliques, des cyanures métalliques complexes, des sulfates métalliques, des pigments de bronze et/ou parmi les pigments colorés à base de mica ou de glimmer, qui sont recouverts d'au moins un oxyde métallique et/ou d'un oxychlorure métallique.

6. Procédé selon l'une des revendications 1 à 5, **caractérisé en ce que** l'agent décolorant est appliqué sur une matière kératinique qui a été colorée par l'application d'au moins un pigment organique, le pigment organique étant de préférence choisi parmi le groupe comprenant le carmin, la quinacridone, la phtalocyanine, le sorgho, des pigments bleus portant les numéros d'index des couleurs CI 42090, CI 69800, CI 69825, CI 73000, CI 74100, CI 74160, des pigments jaunes portant les numéros d'index des couleurs CI 11680, CI 11710, CI 15985, CI 19140, CI 20040, CI 21100, CI 21108, CI 47000, CI 47005, les pigments verts portant les numéros d'index des couleurs CI 61565, CI 61570, CI 74260, des pigments orange portant les numéros d'indice de couleur CI 11725, CI 15510, CI 45370, CI 71105, ainsi que des pigments rouges portant les numéros d'indice de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

7. Procédé selon l'une des revendications 1 à 6, **caractérisé en ce que** l'agent décolorant contient au moins un tensioactif anionique (a) de formule (T-1), 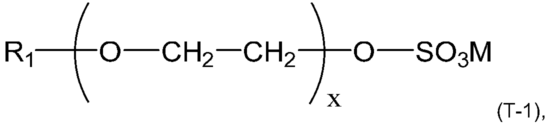 dans laquelle
R₁ représente un groupe alkyle en C₈ à C₃₀, linéaire ou ramifié, saturé ou insaturé,
x représente un nombre entier compris entre 0 et 50, et
M représente un atome d'hydrogène, un ion ammonium (NH₄)₊ ou un équivalent d'un cation mono- ou polyvalent.

8. Procédé selon l'une des revendications 1 à 7, **caractérisé en ce que** l'agent décolorant contient au moins un tensioactif anionique (a) de formule (T-2), 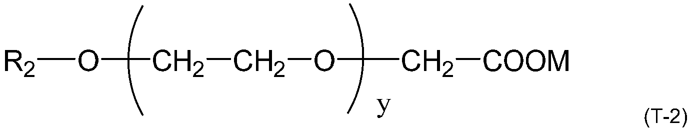 où
R2 représente un groupe alkyle en C₈ à C₃₀, linéaire ou ramifié, saturé ou insaturé,
y représente un nombre entier compris entre 0 et 16,
M représente un atome d'hydrogène ou un métal tel qu'un métal alcalin, notamment le sodium, le potassium, le lithium, un métal alcalino-terreux, notamment ½ magnésium, ½ calcium, ½ zinc, ou un ion ammonium (NH₄⁺).

9. Procédé selon l'une des revendications 1 à 8, **caractérisé en ce que** l'agent décolorant contient - par rapport au poids total de l'agent décolorant - un ou plusieurs tensioactifs anioniques (a) en une quantité totale de 1,0 à 15,0 % en poids, de préférence de 1,5 à 13,0 % en poids, de préférence encore de 3,0 à 12,0 % en poids et, de manière tout particulièrement préférée, de 6,0 à 11,0 % en poids.

10. Procédé selon l'une des revendications 1 à 9, **caractérisé en ce que** l'agent décolorant présente un pH (b) compris entre 1,5 et 3,3, de préférence entre 1,8 et 3,0 et de manière particulièrement préférée entre 2,0 et 3,0.

11. Procédé selon l'une des revendications 1 à 10, **caractérisé en ce que** l'agent décolorant est appliqué sur la matière kératinique colorée et, après un temps de pose de 5 à 60 minutes, de préférence de 5 à 30 minutes, plus préférablement de 5 à 15 minutes et tout particulièrement de préférence de 5 à 10 minutes, est rincé.

12. Procédé de coloration puis de décoloration des cheveux humains, comprenant les étapes suivantes :
(1) Application d'un colorant sur les cheveux, ledit colorant contenant au moins un polymère de silicone à fonction amino et au moins un pigment, tels que définis dans les revendications 1 à 6,
(2) laisser agir l'agent colorant sur les cheveux,
(3) rincer l'agent colorant des cheveux,
(4) appliquer un agent décolorant sur les cheveux, l'agent décolorant étant celui défini dans les revendications 1 à 10,
(5) Laisser agir le produit de post-traitement sur les cheveux,
(6) Rincer le produit de post-traitement des cheveux.

13. Ensemble d'emballage à plusieurs composants (kit de pièces) destiné à la coloration et à la décoloration de la kératine, comprenant, conditionnés séparément les uns des autres :
- un premier récipient contenant un agent colorant, ledit agent colorant contenant au moins un polymère de silicone à fonction amino et au moins un pigment, tels que définis dans les revendications 1 à 6, et
- un deuxième récipient contenant un agent décolorant, ledit agent décolorant étant défini dans les revendications 1 à 10.
